# EUROPEAN PATENT SPECIFICATION

(11) **EP 4 673 115 B1**
(45) Date of publication and mention of the grant of the patent: **09.12.2026**
(21) Application number: 24710822.8
(22) Date of filing: 29.02.2024
(51) Int. Cl.: A61K 9/10, A61K 47/10, A61K 47/36, A61P 5/28, A61P 5/32, A61K 31/58

(54) **LIQUID FORMULATION COMPRISING TRILOSTANE**
FLÜSSIGE ZUBEREITUNG ENTHALTEND TRILOSTAN
FORMULATION LIQUIDE COMPRENANT DU TRILOSTANE

(30) Priority: 02.03.2023 GB 202303094
(43) Date of publication of application: 07.01.2026
(73) Proprietor: Norbrook Laboratories Limited, Newry Down BT35 6QQ (GB)
(72) Inventor: BRANNIGAN, Tim, Newry Down BT35 6QQ (GB); BOYD, Jonathan, Newry Down BT35 6QQ (GB)
(74) Representative: HGF
(86) International application number: PCT/GB2024/050547
(87) International publication number: WO 2024/180339

(56) References cited:
- US-A1- 2007 142 341
- US-A1- 2011 171 307
- CROSBY JESSE ET AL: "Stability of compounded trilostane suspension in cod liver oil", VETERINARY JOURNAL, vol. 228, 2 October 2017 (2017-10-02), pages 15 - 17, XP085251416, ISSN: 1090-0233, DOI: 10.1016/J.TVJL.2017.10.001

## Description

### FIELD OF THE INVENTION

The invention relates to a liquid formulation of trilostane for oral use. The invention further relates to uses of the liquid formulation, methods of treatment involving the liquid formulation and a kit of parts including the liquid formulation.

### BACKGROUND

Trilostane (named 4α,5-epoxy-3,17β-dihydroxy-5α-androst-2-ene-2-carbonitrile) is a synthetic androstane steroid having the structure shown below.

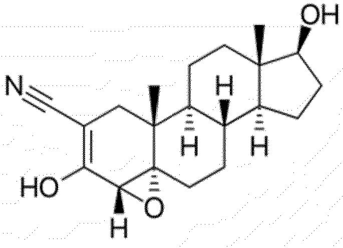

As an inhibitor of 3β-hydroxysteroid dehydrogenase (3β-HSD), trilostane blocks the conversion of Δ⁵-3β-hydroxysteroids into Δ⁴-3-ketosteroids. As such, trilostane inhibits the production of all classes of steroid hormones, including androgens, estrogens, progestogens, glucocorticoids, and mineralocorticoids.

In humans, trilostane has been used to treat Cushing's syndrome, Conn's syndrome and postmenopausal breast cancer, but has now been withdrawn from human use in many countries. Trilostane is, however, still used as a veterinary medicine, primarily to treat Cushing's syndrome in dogs.

Trilostane is a Biopharmaceutical Classification System (BCS) Class Two compound, owning to its low water solubility and high membrane permeability. This can result in significant variability in absorption of the active pharmaceutical ingredient when administered orally.

The approved dosage form of trilostane is a solid dosage form, specifically a hard oral capsule. It is recommended that trilostane is titrated during the initial treatment of disease, starting at a dosage of 2.2 mg/kg to 10.0 mg/kg as required. If the symptoms of hyperadrenocorticism are still present, it is then recommended that the dose is increased after 14 days. As the commercially available product is in the form of a hard capsule at specific dosage strengths, this procedure can be inconvenient for a veterinary surgeon or other end-user owing to the varying sizes in the canine species and the limited dispensability of the drug product. Difficulties may also be encountered if the animal rejects the dosage form.

As can be seen from its structure above, trilostane is a steroidal compound. When dissolved in an aqueous solution, the trilostane may not remain stably dissolved in the solution or, at least, the formulation may not be bioequivalent to the commercially available product, after a prolonged period of time. The poor stability of such solutions or the lack of bioequivalence is problematic commercially. As such, aqueous liquid formulations tend to have an impractically short shelf life, which is a disadvantage, especially in view of the chronicity of Cushing's syndrome.

US 2011/171307 A1 relates to methods of treatment of specific diseases and conditions with specific steroids.

US 2007/142341 A1 relates to steroid compounds for the treatment of an angiotensin II related disease in humans and animals.

CROSBY JESSE ET AL: "Stability of compounded trilostane suspension in cod liver oil", VETERINARY JOURNAL, vol. 228, 2 October 2017, pages 15-17, XP085251416, ISSN: 1090-0233, DOI: 10.1016/J.TVJL. 2017.10.001 describes compounds of trilostane suspended in cod liver oil.

### SUMMARY OF THE INVENTION

The invention provides a liquid formulation. The liquid formulation comprises:
(a) trilostane or a pharmaceutically acceptable salt thereof;
(b) a viscosity modifier;
(c) an organic co-solvent; and
(d) water.

The organic co-solvent has at least one hydroxy group. The organic solvent is water-soluble or water-miscible.

The inventors have surprisingly been able to develop a stable liquid formulation of trilostane that is suitable for oral administration and which is bioequivalent to the approved solid dosage form (e.g. the hard capsules). The liquid formulation of the invention can be stored over a prolonged period, with the trilostane remaining efficacious (e.g. maintains bioequivalence) over a pharmaceutically relevant timeframe. The liquid formulation is also associated with several advantages relating to the way it can be manufactured industrially.

The liquid formulation of the invention can improve the prognosis and outcome of Cushing's disease and syndrome, particularly for veterinary uses. The formulation of the active pharmaceutical ingredient (API) as a liquid dosage form is easier to administer to animals than the approved solid formulation. It can also be administered in the correct dosage amount more precisely. These advantages offer a significant improvement for the administration of trilostane to both the animal and its owner.

An aspect of the invention is a liquid formulation for use in therapy and/or for use as a medicament. The liquid formulation for these uses is a liquid formulation in accordance with the invention.

The invention further provides a liquid formulation for use in the treatment or prevention of pituitary-dependent hyperadrenocorticism or adrenal-dependent hyperadrenocorticism (Cushing's disease and syndrome). The liquid formulation is a liquid formulation in accordance with the invention.

The invention also provides a method of treating or preventing pituitary-dependent hyperadrenocorticism or adrenal-dependent hyperadrenocorticism (Cushing's disease and syndrome) in a subject comprising orally administering a liquid formulation to the subject. The liquid formulation is a liquid formulation in accordance with the invention.

A further aspect of the invention is a kit of parts comprising (a) the liquid formulation and (b) a syringe. The liquid formulation is a liquid formulation in accordance with the invention.

### BRIEF DESCRIPTION OF FIGURES

The invention is further described hereinafter with reference to the accompanying figures.
Figure 1 is a graph showing a comparison between plasma trilostane concentrations in dogs after oral administration of Formulation No. 1 in Example 1 and a Reference Product (Vetoryl^{™} capsules).
Figure 2 is a graph showing a comparison between plasma trilostane concentrations in dogs after oral administration of Formulation No. 2 in Example 1 and a Reference Product (Vetoryl^{™} capsules).

### DETAILED DESCRIPTION

The invention provides a liquid formulation. The liquid formulation is for oral administration, preferably for direct oral administration or for oral administration with food. It is not necessary to mix the liquid formulation with an excipient or to dilute it in water before administration.

The liquid formulation may be ingested with food. For example, the liquid formulation may be co-administered or mixed with food. This form of oral administration may be convenient when administering the formulation to the subject.

BCS II drug compounds, such as trilostane, have poor water solubility with high permeability. As such, these compounds are formulated as immediate release solid dosage forms to ensure the maximum amount of the drug is available for dissolution.

When re-formulating a regulatory approved drug product into a different dosage form, it is desirable for the new formulation to be bioequivalent to the pioneer reference product. This can be difficult when re-formulating a drug product in the form of capsules as a liquid dosage form because of the inherent delay associated with the disintegration and subsequent wetting of the drug particulates in the solid dosage form.

The liquid formulation of the invention improves the dosing accuracy and patient acceptability, while maintaining the safety and efficacy of the administered trilostane. The liquid formulation may be bioequivalent to the approved solid formulation.

The liquid formulation comprises trilostane or a pharmaceutically acceptable salt thereof. The pharmaceutically acceptable salt of trilostane is preferably a veterinary pharmaceutically acceptable salt.

The trilostane or the pharmaceutically acceptable salt of trilostane is the active pharmaceutical ingredient (API) of the formulation.

Trilostane has two hydroxy groups. One or more of these hydroxy groups may be deprotonated to form a pharmaceutically acceptable salt of trilostane.

The pharmaceutically acceptable salt of trilostane may, for example, be an alkali metal salt; an alkaline earth metal salt; an ammonium salt (NH₄⁺), a primary ammonium cation (RNH₃⁺), a secondary ammonium cation (R₂NH₂⁺), a tertiary ammonium cation (R₃NH⁺), a quarternary ammonium salt (R₄N⁺); or a salt with an organic base which affords a physiologically acceptable cation. The alkali metal salt of trilostane may be a lithium, a sodium or a potassium salt of trilostane. The alkaline earth metal salt of trilostane may be a calcium or a magnesium salt of trilostane. The organic base (e.g. for forming the pharmaceutically acceptable salt) may be piperidine, morpholine, tris(2-hydroxyethyl)amine, methylamine, dimethylamine, trimethylamine, as well as non-toxic ammonium, quaternary ammonium and amine cations including, but not limited to, ammonium, tetramethylammonium, tetraethylammonium, lysine, arginine, benzathine, choline, tromethamine, diolamine, glycine, meglumine, olamine and the like.

The amount of active pharmaceutical ingredient used herein refers to the amount of trilostane per se in the formulation. Any reference to an amount of trilostane as used herein in connection with a pharmaceutically acceptable salt of trilostane is to be understood as referring to an equivalent molar amount of the pharmaceutically acceptable salt of trilostane.

In general, any amount as used herein (e.g. expressed as a % w/v) is expressed in terms of the liquid formulation as a whole, unless the context indicates otherwise.

Typically, the liquid formulation comprises trilostane. It is preferred that the aqueous liquid formulation comprises trilostane as the only (e.g. sole) active pharmaceutical ingredient for treating or preventing Cushing's disease or Cushing's syndrome, more preferably the only active pharmaceutical ingredient per se.

The liquid formulation generally comprises a total amount of about 0.1 % w/v to about 10.0 % w/v of trilostane, preferably a total amount of about 0.2 % w/v to about 5.0 % w/v of trilostane, more preferably about 0.5 % w/v to about 3.0 % w/v of trilostane, even more preferably about 1.0 % w/v to about 2.5 % w/v of trilostane. As mentioned above, the liquid formulation may alternatively comprise a molar equivalent amount of a pharmaceutically acceptable salt of trilostane in these ranges.

Typically, the liquid formulation comprises a total amount of about 5.0 mg/mL to about 50.0 mg/mL of trilostane, preferably about 10.0 mg/mL to about 30.0 mg/mL of trilostane, more preferably about 15.0 mg/mL to about 25.0 mg/mL of trilostane (e.g. about 20.0 mg/mL of trilostane).

The liquid formulation of the invention comprises a dispersion of trilostane or a pharmaceutically acceptable salt thereof. It is preferred that the liquid formulation comprises a suspension of trilostane or a pharmaceutically acceptable salt thereof.

The liquid formulation may be in the form of a solution, a suspension, a gel or a paste. When the liquid formulation is a gel, then it may be a hydrogel. It is preferred that the liquid formulation is in the form of a solution or a suspension.

Typically, the liquid formulation is a homogenous mixture.

In general, particles of trilostane are dispersed (or preferably suspended) in a liquid (e.g. the water and/or organic co-solvent of the liquid formulation) when manufacturing the liquid formulation.

The particles of trilostane typically have a D₅₀ of 25 µm or less and a D₉₀ of 60 µm or less, preferably a D₅₀ of 20 µm or less and a D₉₀ of 50 µm or less, more preferably a D₅₀ of less than 20 µm and a D₉₀ of less than 50 µm. The particle size of the trilostane is determined using conventional particle size measurement techniques in the pharmaceuticals industry. The particle sizes and the distribution of particle sizes may be measured by laser diffraction and by applying a Mie analysis (e.g. by using a Mastersizer 3000 particle size analyzer by Malvern Panalytical).

The liquid formulation comprises a viscosity modifier. The viscosity modifier may be selected from the group consisting of a polysaccharide, a cellulose or a cellulose derivative, a hydrophilic polymer, a silica derivative and a mixture of two or more thereof. It is preferred that the viscosity modifier is selected from the group consisting of a polysaccharide, a cellulose or a cellulose derivative, a hydrophilic polymer and a mixture of two or more thereof. More preferably, the viscosity modifier is either (a) a polysaccharide, a cellulose or cellulose derivative or a mixture of two or more thereof, or (b) a polysaccharide, a hydrophilic polymer or a mixture of two or more thereof. Even more preferably, the viscosity modifier is a mixture of (i) a polysaccharide and (ii) a cellulose or cellulose derivative.

For the avoidance of doubt, the polysaccharide is different (e.g. in structure and/or chemical composition) to the cellulose and cellulose derivative. It is preferred that the polysaccharide is not a cellulose or cellulose derivative. The polysaccharide may be a non-cellulosic polysaccharide.

When the viscosity modifier includes a polysaccharide, then the polysaccharide may be selected from the group consisting of xanthan gum, a carrageenan, tragacanth gum, guar gum, agar, pectin and a mixture of two or more thereof. It is preferred that the polysaccharide is xanthan gum or pectin, more preferably the polysaccharide is xanthan gum.

Examples of suitable xanthan gums that can be used include Rhodigel 80^{™}, Rhodigel 23^{™}, Keltrol^{™}, Keltrol^{™} F, Keltrol^{™} T, Keltrol^{™} TF, Keltrol^{™} 1000 and Merezan^{™}.

When the viscosity modifier includes or consists of a polysaccharide, then the liquid formulation typically has a total amount of the polysaccharide of about 0.1 to about 1.5 % w/v, preferably from about 0.2 to 1.0 % w/v, more preferably from about 0.4 to 0.8 % w/v.

It is preferred that the liquid formulation includes xanthan gum (e.g. as a viscosity modifier that is a polysaccharide) in a total amount of about 0.1 to about 1.5 % w/v, preferably from about 0.2 to 1.0 % w/v, more preferably from about 0.4 to 0.8 % w/v.

When the viscosity modifier includes a cellulose or cellulose derivative, then the cellulose or the cellulose derivative may be selected from the group consisting of cellulose; a carboxymethylcellulose or a salt thereof; ethyl cellulose; methyl cellulose; a cellulose ether; a hydroxyethyl cellulose or a salt thereof; a hydroxypropyl cellulose or a salt thereof; a hydroxypropyl methylcellulose (e.g. hydroxypropyl methyl cellulose acetate succinate) or a salt thereof; and a mixture of two or more thereof. It is preferred that the cellulose or cellulose derivative is selected from the group consisting of cellulose; a carboxymethylcellulose or a salt thereof; and a mixture of two or more thereof. More preferably, the cellulose or cellulose derivative is a mixture of cellulose and carboxymethylcellulose or a salt thereof, particularly a mixture of cellulose and carboxymethylcellulose sodium.

Any reference to a "salt thereof" used in the context of a cellulose or cellulose derivative relates to a pharmaceutically acceptable salt, particularly a veterinary pharmaceutical acceptable salt. It is preferred that the "salt thereof" is a sodium or potassium salt, more preferably a sodium salt.

The cellulose or the cellulose derivative may be a microcrystalline cellulose or a microcrystalline cellulose derivative. When the viscosity modifier includes cellulose, then it is preferred that the cellulose is a microcrystalline cellulose.

When the viscosity modifier includes or consists of a cellulose and/or a cellulose derivative, then the liquid formulation typically has a total amount of the cellulose and/or the cellulose derivative of about 0.05 to about 1.5 % w/v, preferably from about 0.1 to about 1.0 % w/v, more preferably from about 0.2 to about 0.6 % w/v.

It is preferred that the liquid formulation includes cellulose (e.g. microcrystalline cellulose) and carboxymethylcellulose, particularly carboxymethylcellulose sodium, (e.g. as a viscosity modifier that is a cellulose or a cellulose derivative) in a total amount of about 0.05 to about 1.5 % w/v, preferably from about 0.1 to about 1.0 % w/v, more preferably from about 0.2 to about 0.6 % w/v.

Generally, when the liquid formulation, particularly the viscosity modifier, includes cellulose and a cellulose derivative, such as microcrystalline cellulose and carboxymethylcellulose or a salt thereof, then the cellulose and the cellulose derivative are included in a ratio by mass of about 25:1 to about 1:5, preferably a ratio by mass of about 20:1 to about 1:1, more preferably a ratio by mass of about 15:1 to about 5:1, such as about 12:1 to about 6:1.

The mass of the cellulose is, in general, greater than the mass of the cellulose derivative in the liquid formulation.

When the viscosity modifier includes a hydrophilic polymer, then the hydrophilic polymer may be selected from the group consisting of polyvinylpyrrolidone, a polyvinylpyrrolidone-vinyl acetate copolymer, a polyvinyl acetate, a polyvinyl acetate-acrylate, a polyethylene glycol (PEG), a poloxamer and a mixture of two or thereof., preferably the hydrophilic polymer is polyvinylpyrrolidone. It is preferred that the hydrophilic polymer is polyvinylpyrrolidone.

When the viscosity modifier includes or consists of a hydrophilic polymer, then the liquid formulation typically has a total amount of the hydrophilic polymer of about 0.1 to about 1.5 % w/v, preferably from about 0.2 to about 1.0 % w/v, more preferably from about 0.3 to about 0.7 % w/v.

**It** is preferred that the liquid formulation includes polyvinylpyrrolidone (e.g. as a viscosity modifier that is a hydrophilic polymer) in a total amount of about 0.1 to about 1.5 % w/v, preferably from about 0.2 to about 1.0 % w/v, more preferably from about 0.3 to about 0.7 % w/v.

When the viscosity modifier includes a silica derivative, then the silica derivative may be a silica having a surface with methylated silanol groups.

When the viscosity modifier includes or consists of a silica derivative polymer, then the liquid formulation typically has a total amount of the silica derivative of about 0.1 to about 5.0 % w/v, preferably from about 0.2 to about 2.5 % w/v, such as from about 0.5 to 2.0 % w/v.

In a preferred liquid formulation of the invention, the viscosity modifier includes or consists of:
(i) a polysaccharide in a total amount of about 0.1 to about 1.5 % w/v; and
(ii) a cellulose and a cellulose derivative in a total amount of about 0.05 to about 1.5 % w/v.

In (i) above, the polysaccharide is preferably xanthan gum.

In (ii) above, the cellulose is preferably microcrystalline cellulose and/or the cellulose derivative is preferably carboxymethylcellulose. The microcrystalline cellulose and carboxymethylcellulose are preferably included in a ratio by mass of about 25:1 to about 1:5, more preferably a ratio by mass of about 20:1 to about 1:1, especially a ratio by mass of about 15:1 to about 5:1, and even more preferably a ratio by mass of about 12:1 to about 6:1.

In an alternative preferred liquid formulation of the invention, the viscosity modifier includes or consists of:
(i) a polysaccharide in a total amount of about 0.1 to about 1.5 % w/v; and
(ii) a hydrophilic polymer in a total amount of about 0.1 to about 1.5 % w/v.

In (i) above, the polysaccharide is preferably xanthan gum.

In (ii) above, the hydrophilic polymer is preferably polyvinylpyrrolidone.

For the avoidance of doubt, the total amounts above are expressed in terms of the % w/v of the liquid formulation.

It is believed that the invention utilises a principle of polymeric matrix drug release involving the viscosity modifier, such as a polysaccharide, a cellulose or cellulose derivative or a hydrophilic polymer. The polymer can form a matrix within solution, in which the drug compound is dispersed. By modifying the concentration of the polymer, the rates of diffusion and erosion of the matrix from which the drug compound is released can be modified. The introduction of certain, other rheological modifying agents is believed to have an impact on the structure of the polymer matrix, which can further modify the drug release profile of the matrix. These features permit the manufacture of a liquid formulation, which has been demonstrated using *in vitro* and *in vivo* testing methods to meet bioequivalence requirements.

The liquid formulation of the invention comprises water. Thus, the liquid formulation may be regarded as being an aqueous liquid formulation. Water is used a solvent in the liquid formulation.

The liquid formulation of the invention also comprises an organic co-solvent. The organic co-solvent is water-soluble or water-miscible. As the liquid formulation contains water as a solvent, then the organic co-solvent should be able to form a homogeneous composition, such as a liquid or a gel, when mixed with water.

The requirement for the organic co-solvent to be water-soluble or water-miscible refers to the solubility or miscibility of the amount of organic co-solvent used in the liquid formulation at a temperature of about 20 °C (e.g. at standard atmospheric pressure, 1013.25 mbar).

The organic co-solvent may be a solid or a liquid, typically at a temperature of about 20 °C (e.g. at standard atmospheric pressure, 1013.25 mbar). It is preferred that the organic co-solvent is a liquid.

The term "water-soluble" as used herein refers to an organic co-solvent, typically an organic co-solvent that is a solid (e.g. at a temperature of about 20 °C and at standard atmospheric pressure, 1013.25 mbar), that is capable of being dissolved in water at the temperature of about 20 °C (e.g. at standard atmospheric pressure, 1013.25 mbar), preferably in an amount that is used in the liquid formulation. The term "water-miscible" as used herein refers to an organic co-solvent, typically an organic co-solvent that is a liquid (e.g. at a temperature of about 20 °C and at standard atmospheric pressure, 1013.25 mbar), that is capable of being dissolved in water at the temperature of about 20 °C (e.g. at standard atmospheric pressure, 1013.25 mbar), preferably in an amount that is used in the liquid formulation.

Typically, the organic co-solvent has a partition coefficient (P) where log *P*_{hex/wat} is less than 1 (e.g. at a temperature of about 20 °C and at standard atmospheric pressure, 1013.25 mbar). More preferably, log *P*_{hex/wat} is less than 0. *P*_{hex/wat} is the partition coefficient in a biphasic system of hexane and water.

The organic co-solvent has at least one hydroxy group (-OH). Typically, the organic co-solvent is hydrophilic.

The organic co-solvent may be an alcohol, preferably a non-toxic alcohol. The term "alcohol" as used herein has its conventional meaning and refers to an organic compound in which a hydroxy group (-OH) is bonded to a carbon atom. The term "non-toxic" as used herein the context of an alcohol refers to an alcohol that is not toxic when ingested by the subject. Examples of toxic alcohols include methanol, ethylene glycol and isopropyl alcohol.

The alcohol, including the non-toxic alcohol, may be a monohydric alcohol having 2 to 6 carbon atoms, a polyhydric alcohol (e.g. a polyol) having 2 to 8 carbon atoms, a polyethylene glycol or a poloxamer. It is preferred that the alcohol, particularly the non-toxic alcohol, is a monohydric alcohol having 2 to 6 carbon atoms, a polyol having 2 to 8 carbon atoms or a polyethylene glycol. More preferably, the alcohol, particularly the non-toxic alcohol, is a monohydric alcohol having 2 to 6 carbon atoms or a polyol having 2 to 8 carbon atoms. Most preferred is a polyol having 2 to 8 carbon atoms.

For the avoidance of doubt, the term "polyhydric alcohol" or "polyol" as used herein includes a dihydric alcohol and a sugar alcohol. It is preferred that the polyhydric alcohol or polyol has 2, 3 or 4 hydroxy groups, more preferably 2 or 3 hydroxy groups.

It is preferred that the monohydric alcohol has 2 to 4 carbon atoms. More preferably, the monohydric alcohol is ethanol or n-butanol, especially ethanol.

It is preferred that the polyol has 2 to 6 carbon atoms, more preferably 2 to 4 carbon atoms. The polyol may be propylene glycol, 1,3-propanediol, glycerol or a mixture of two or more thereof. The polyol may be glycerol. Propylene glycol is most preferred.

The polyethylene glycol may be a PEG 300 to a PEG 1500 (e.g. a PEG 300, a PEG 400, a PEG 600, a PEG 1000 or a PEG 1450). It is preferred that the polyethylene glycol is a PEG 300 to a PEG 600.

The poloxamer may be poloxamer 124, poloxamer 184, poloxamer 188, poloxamer 338 or poloxamer 407. It is preferred that the poloxamer is poloxamer 188 or poloxamer 407, more preferably poloxamer 188.

When the viscosity modifier includes polyethylene glycol and/or a poloxamer (e.g. as a hydrophilic polymer), then it is preferred that the organic co-solvent is a different ingredient to the viscosity modifier. Thus, the polyethylene glycol as the viscosity modifier may be a different polyethylene glycol to that used for the organic co-solvent.

Typically, the organic co-solvent may be a monohydric alcohol having 2 to 6 carbon atoms or a polyhydric alcohol (e.g. a polyol) having 2 to 8 carbon atoms when the viscosity modifier includes polyethylene glycol and/or a poloxamer.

In general, the liquid formulation has a total amount of the organic co-solvent of from about 2.5 to about 60.0 % w/v (e.g. about 2.5 to about 25.0 % w/v or about 30.0 to about 60.0 % w/v), preferably from about 5.0 to 55.0 % w/v (e.g. about 5.0 to about 20.0 % w/v or about 40.0 to about 55.0 % w/v), more preferably 7.5 to 25.0 % w/v.

In a preferred liquid formulation of the invention, the liquid formulation includes:
(i) a polysaccharide in a total amount of about 0.1 to about 1.5 % w/v;
(ii) a cellulose and a cellulose derivative in a total amount of about 0.05 to about 1.5 % w/v; and
(iii) an organic co-solvent in a total amount of about 2.5 to about 25.0 % w/v, preferably about 5.0 to about 20.0 % w/v.

In (i) above, the polysaccharide is preferably xanthan gum.

In (ii) above, the cellulose is preferably microcrystalline cellulose and/or the cellulose derivative is preferably carboxymethylcellulose. The microcrystalline cellulose and carboxymethylcellulose are preferably included in a ratio by mass of about 25:1 to about 1:5, more preferably a ratio by mass of about 20:1 to about 1:1, especially a ratio by mass of about 15:1 to about 5:1, and even more preferably a ratio by mass of about 12:1 to about 6:1.

In (iii) above, the organic co-solvent is preferably propylene glycol.

In an alternative preferred liquid formulation of the invention, the liquid formulation includes:
(i) a polysaccharide in a total amount of about 0.1 to about 1.5 % w/v;
(ii) a hydrophilic polymer in a total amount of about 0.1 to about 1.5 % w/v; and
(iii) an organic co-solvent in a total amount of about 30.0 to about 60.0 % w/v, preferably about 40.0 to about 55.0 % w/v.

In (i) above, the polysaccharide is preferably xanthan gum.

In (ii) above, the hydrophilic polymer is preferably polyvinylpyrrolidone.

In (iii) above, the organic co-solvent is preferably glycerol.

The liquid formulation comprises water. As a general feature, the liquid formulation comprises a balance amount of water.

Typically, the liquid formulation comprises a total amount of water of at least about 35.0 % w/v, preferably at least about 40.0 % w/v, more preferably at least about 45.0 % w/v.

Water may be the main solvent in the liquid formulation. Thus, the liquid formulation may comprise a total amount of water of at least about 50.0 % w/v, such as at least about 55.0 % w/v, preferably at least about 60.0 % w/v, more preferably at least about 75.0 % w/v.

Generally, the liquid formulation comprises a total amount of water of about 35.0 % w/v to about 90.0 % w/v, preferably about 45.0 % w/v to about 85.0 % w/v.

The ratio by mass of water to the organic co-solvent may be from about 10:1 to about 1:2, preferably from 8:1 to 2:3, more preferably 15:2 to 1:1.

The liquid formulation may further comprise one or more further excipients. For example, the liquid formulation may comprise an excipient selected from a preservative, a buffering agent, an antifoaming agent, a flavouring agent, a sweetener, a colouring agent and a combination of two or more thereof.

In general, the liquid formulation may further comprise a preservative. The preservative can protect the liquid formulation from microbial contamination.

The preservative may, for example, be selected from the group consisting of benzoic acid or a sodium or potassium salt thereof; scorbic acid or a sodium or potassium salt thereof; a phenolic compound (e.g. phenol, meta-cresol, chlorocresol); a quaternary ammonium compound (e.g. benzylalkonium chloride, benzethonium chloride); a hydroxy benzoate ester (e.g. methyl hydroxybenzoate, ethyl hydroxybenzoate, propyl hydroxybenzoate, butyl hydroxybenzoate); a paraben (e.g. methyl, ethyl, propyl or butyl paraben); and a mixture of two or more thereof . In a preferred embodiment, the preservative is a hydroxy benzoate ester.

The preservative can be present in a total amount of from about 0.02 to about 5.0 % w/v of the liquid, preferably about 0.05 to about 3.0 % w/v, most preferably in an amount of about 0.10 to about 1.0 % w/v.

The liquid formulation may comprise a buffering agent. The buffering agent is included to regulate the pH of the liquid formulation.

The buffering agent may include a weak acid (e.g. citric acid) and/or a conjugate base of a strong acid (e.g. a phosphate salt, such as disodium phosphate, and/or a dihydrogen phosphate salt).

In one example, it is preferred that the buffering agent includes citric acid.

The liquid formulation may comprise a buffering agent in a total amount of from about 0.1 to about 5.0 % w/v of the liquid, preferably about 0.5 to about 3.0 % w/v, most preferably in an amount of about 1.0 to about 2.0 % w/v.

The liquid formulation may comprise an antifoaming agent. Such antifoaming agents are well known in the art.

The antifoaming agent can be present in a total amount of from about 0.01 to about 1.5 % w/v of the liquid, preferably about 0.10 to about 1.0 % w/v, most preferably in an amount of about 0.15 to about 0.50 % w/v.

The liquid formulation may comprise a flavouring agent. The flavouring agent is selected to make the liquid formulation more palatable to the subject.

The flavouring agent may be an apple flavour, a cherry flavour, a raspberry flavour, a blackcurrant flavour, a strawberry flavour, a honey flavour, a chocolate flavour, a caramel flavour, a mint cool flavour, an aniseed flavour, a beef flavour, a pork flavour or a chicken flavour. It is preferred that the flavouring agent is an aniseed flavour. It is also preferred that the flavouring agent is a honey flavour.

The flavouring agent can be present in an amount of from about 0.01 to about 3.0 % w/v of the liquid formulation, preferably about 0.1 to about 1.5% w/v.

The liquid formulation may further comprise a sweetener. The sweetener may be a bulk sweetener or an intense sweetener.

The bulk sweetener may be selected from the group consisting of sorbitol, mannitol, fructose, sucrose, maltose, isomalt, glucose, hydrogenated glucose syrup, xylitol and a mixture of two or more thereof. It is preferred that the sweetener is sorbitol.

The bulk sweetener can be present in an amount of from about 1.0 to about 10.0 % w/v of the liquid formulation, preferably from about 3.0 to about 6.0 % w/v, more preferably about 5.0 to about 6.0 % w/v.

The intense sweetener may be selected from the group consisting of saccharin, aspartame, acesulfame, cyclamate, alitame, taumatin, a dihydrochalcone sweetener, monellin, neohesperidin, neotame, stevioside, sucralose, the pharmaceutically acceptable salts thereof such as sodium or calcium saccharin, acesulfame potassium or sodium cyclamate, and mixtures of two or more thereof. It is preferred that the intense sweetener is sodium saccharin.

The intense sweetener can be present in an amount of from about 0.01 to about 1.0 % w/v of the liquid formulation, preferably about 0.03 to about 0.50 % w/v, more preferably about 0.05 to about 0.25 % w/v.

A colouring agent may be included in the liquid formulation. The colouring agent may provide an appealing colour.

Examples of suitable colouring agents include FD&C Red #40, FD&C Blue #1 and FD&C Red #33.

The invention also provides a liquid formulation for use in therapy and/or for use as a medicament.

The present invention provides a liquid formulation for use in a method of treatment of the human or animal body by therapy, preferably the animal body by therapy.

The term "animal" as used herein refers to a non-human animal. It is preferred that the animal is a dog or a cat, preferably a dog.

The invention further provides a liquid formulation for use in the treatment or prevention of pituitary-dependent hyperadrenocorticism or adrenal-dependent hyperadrenocorticism, preferably in a non-human animal, such as a dog or a cat, more preferably a dog.

Also provided by the invention is the use of a liquid formulation for the manufacture of a medicament for the treatment or prevention of pituitary-dependent hyperadrenocorticism or adrenal-dependent hyperadrenocorticism, preferably in a non-human animal such as a dog or a cat, more preferably a dog.

Pituitary-dependent hyperadrenocorticism is also known as Cushing's disease. Adrenal-dependent hyperadrenocorticism is also referred to as Cushing's syndrome.

The invention further provides a method of treating or preventing pituitary-dependent hyperadrenocorticism or adrenal-dependent hyperadrenocorticism in a subject. The subject is preferably in a non-human animal, such as a dog or a cat, more preferably a dog.

The method comprises administering the liquid formulation to the subject. The liquid formulation is to be orally administered to the subject (e.g. the recipient subject).

The liquid formulation may be orally administered directly into the mouth of the subject, such as by injecting or squirting the liquid formulation into the mouth using, for example, a syringe or a dispenser, such as described herein.

The liquid formulation may be orally administered by co-administration or mixing of the liquid formulation with food.

The trilostane or pharmaceutically acceptable salt thereof is present in a "unit dose volume" of the liquid formulation, typically in a therapeutically effect amount. The therapeutically effective amount is an amount that produces the desired therapeutic response upon oral administration. In determining such amounts, the dosage regimen, the age and weight of the subject, and other factors must be considered, as known in the art.

As used herein a "unit dose volume" of the liquid formulation is a convenient volume for dosing the formulation to a recipient subject. The dosing directions provide instructions as to the amount of trilostane that should be taken, which will be multiple of the unit dose volume, depending on, for example, the age or weight of the recipient subject.

The liquid formulation is administered in such an amount that a single daily dosage comprises from about 2 mg/kg to about 10 mg/kg of trilostane (per kg of body weight).

Dosing of the liquid formulation can be done using an appropriate metering system such as, for example a calibrated syringe, or a pre-filled dispenser that can deliver calibrated amounts of the liquid formulation.

The invention further provides a kit of parts comprising (a) the liquid formulation and (b) a syringe, preferably a calibrated syringe.

The term "about" as used herein in conjunction with a measurable value, such as an amount or a period of time, encompasses reasonable variations of the value, for instance, to allow for experimental error in the measurement of said value.

As used herein, the term "comprises" has an open meaning, which allows other, unspecified features to be present. This term embraces, but is not limited to, the semi-closed term "consisting essentially of" and the closed term "consisting of". Unless the context indicates otherwise, the term "comprises" may be replaced with either "consisting essentially of" or "consists of". The term "consisting essentially of" may be replaced with "consists of".

### EXAMPLES

The invention is further illustrated by the following examples which should not be construed as a limiting.

### Example 1

### Formulations

The following formulations were prepared by conventional methods.

**Table 1**

| Ingredient | Function | Formulation No. [% w/v] | |
|---|---|---|---|
| | | 1 | 2 |
| Trilostane | API | 2.0 | 0.5 |
| Propylene glycol | Co-solvent | 12.5 | - |
| Glycerol | Co-solvent | - | 50.52 |
| Xanthan Gum | Viscosity modifier | 0.6 | 0.8 |
| Microcrystalline Cellulose | Viscosity modifier | 0.345-0.367 | - |
| Carboxymethylcellulose sodium | Viscosity modifier | 0.033-0.055 | |
| Polyvinylpyrrolidone (PVP) | Viscosity modifier | - | 0.5 |
| Methyl hydroxybenzoate | Preservative | 0.1 | - |
| Propyl hydroxybenzoate | Preservative | 0.04 | - |
| Disodium Phosphate | Buffering agent | - | 0.2 |
| Sodium Dihydrogen Phosphate | Buffering agent | - | 0.9 |
| Citric Acid | Buffering agent | - | 0.3 |
| Antifoam MS | Antifoaming agent | - | 0.2 |
| Purified water | Vehicle | Q.S. 100 mL | Q.S. 100 mL |

The formulations in Table 1 and the trilostane met the criteria shown in Table 2.

**Table 2**

| | | |
|---|---|---|
| Criterion | Acceptance | |
| Appearance | White to off-white/yellow suspension | |
| pH | 5 to 7 | |
| Viscosity | Shear rate (1/s) | Range (mPa.S) |
| | 5.1 | 1800 - 2400 |
| | 19.1 | 550-800 |
| | 35.1 | 250-550 |
| Particle Size Distribution | Fraction | Range (µm) |
| | D₅₀ | < 10 |
| | D₉₀ | < 20 |
| | D₉₉ | < 50 |

Observations of the formulations and tests with an assay showed that the formulations remained dissolved and were stable over a period of at least 6 months.

### Results

The pharmacokinetics of the formulations in Table 1 were compared to the approved Reference Product, namely Vetoryl^{™} capsules, which is a solid dosage form.

Plasma levels of trilostane in dogs were determined following oral administration of the 2 test formulations from Table 1 above and the Reference Product (Vetoryl^{™} capsules). Each test product was compared to the Reference Product in a four period fully replicated cross-over design. Each animal received one product per period at a nominal dose rate of 60 mg trilostane per administration. Blood samples were collected at designated timepoints pre- and post treatment and analysed to determine trilostane plasma concentrations.

Pharmacokinetic parameters AUC_{0-Last} (area under the concentration/time curve to the last quantifiable timepoint), Cₘₐₓ (the maximum concentration) were determined to investigate the rate and extent of trilostane absorption for each formulation in comparison to the Reference Product.

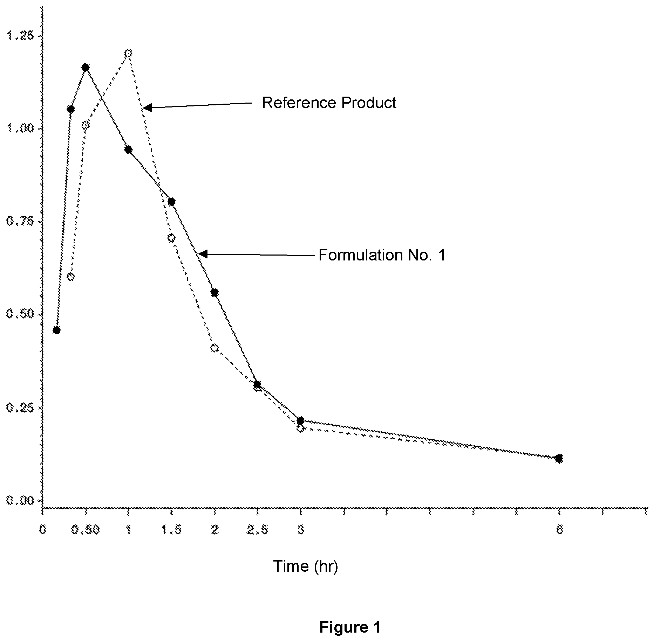

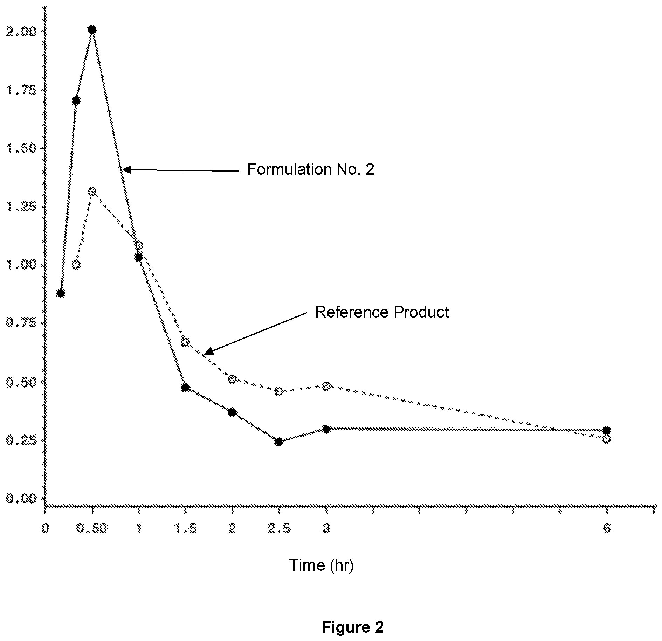

Figure 1 shows the plasma concentrations (y-axis [ng/mL]) in dogs over time of trilostane after administration of Formulation No. 1 in Table 1 above or Vetoryl^{™} capsules. Figure 2 shows the plasma concentrations (y-axis [ng/mL]) in dogs over time of trilostane after administration of Formulation No. 2 in Table 1 above or Vetoryl^{™} capsules.

The results from the bioequivalence studies are shown in Table 3 in relation to the Reference Product.

**Table 3**

| Formulation No. | Parameter | Ratio of Geometric Means |
|---|---|---|
| 1 | AUC_{0-Last} | 103.5 % |
| | Cₘₐₓ | 108.9 % |
| 2 | AUC_{0-Last} | 91.6 % |
| | Cₘₐₓ | 124.5 % |

The value of 103.5 % for the ratio of AUC_{0-Last} for Formulation No. 1 relative to the Reference Product demonstrates similar drug plasma concentrations following administration with both products. The value of 108.9 % for the ratio of concentration max (Cₘₐₓ) relative to the Reference Product demonstrates that a similar maximum peak concentration is achieved after administration with both products. The AUC_{0-Last} and Cₘₐₓ for the second test product, Formulation No. 2, was showed lower similarity than Formulation No. 1 in comparison to the Reference Product. The results indicate that Formulation No. 1 is similar in rate and extent of absorption to demonstrate bioequivalence.

Further dissolution studies were performed using Formulation No. 1. The amounts of propylene glycol, xanthan gum, microcrystalline cellulose and carboxymethylcellulose sodium were varied and the data showed that good dissolution was obtained.

All headings and sub-headings are used herein for convenience only and should not be construed as limiting the invention in any way.

The use of any and all examples, or exemplary language (e.g. "such as") provided herein, is intended merely to better illuminate the invention and does not pose a limitation on the scope of the invention unless otherwise paragraphed. No language in the specification should be construed as indicating any non-paragraphed element as essential to the practice of the invention.

This invention includes all modifications and equivalents of the subject matter recited in the paragraphs appended hereto as permitted by applicable law.

## Claims

1. A liquid formulation comprising:
(a) trilostane or a pharmaceutically acceptable salt thereof;
(b) a viscosity modifier;
(c) an organic co-solvent; and
(d) water;
wherein the organic co-solvent has at least one hydroxy group, and is water-soluble or water-miscible.

2. The liquid formulation according to claim 1, wherein the viscosity modifier is selected from the group consisting of a polysaccharide, a cellulose or a cellulose derivative, a hydrophilic polymer, a silica derivative, and a mixture of two or more thereof, optionally wherein the viscosity modifier includes a polysaccharide selected from the group consisting of xanthan gum, a carrageenan, tragacanth gum, guar gum, agar and a mixture of two or more thereof, preferably the polysaccharide includes xanthan gum.

3. The liquid formulation according to claim 1 or 2, wherein the viscosity modifier includes a polysaccharide, and the liquid formulation has a total amount of the polysaccharide of from 0.1 to 1.5 % w/v, preferably from 0.2 to 1.0 % w/v, more preferably from 0.4 to 0.8 % w/v.

4. The liquid formulation according to any one of claims 1 to 3, wherein the viscosity modifier includes a cellulose or cellulose derivative selected from the group consisting of cellulose; a carboxymethylcellulose or a salt thereof; ethyl cellulose; methyl cellulose; a cellulose ether; a hydroxyethyl cellulose or a salt thereof; a hydroxypropyl cellulose or a salt thereof; a hydroxypropyl methylcellulose or a salt thereof; and a mixture of two or more thereof,
preferably, wherein the cellulose is microcrystalline cellulose.

5. The liquid formulation according to any one of claims 1 to 4, wherein the viscosity modifier includes a cellulose and/or a cellulose derivative, wherein:
the liquid formulation has a total amount of the cellulose and/or the cellulose derivative of 0.05 to 1.5 % w/v, preferably from 0.1 to 1.0 % w/v, more preferably from 0.2 to 0.6 % w/v; and/or
the viscosity modifier includes a cellulose and a cellulose derivative in a ratio by mass of 25:1 to 1:5, preferably a ratio by mass of 15:1 to 5:1.

6. The liquid formulation according to any one of claims 1 to 5, wherein the viscosity modifier includes a hydrophilic polymer wherein;
the hydrophilic polymer is selected from the group consisting of polyvinylpyrrolidone, a polyvinylpyrrolidone-vinyl acetate copolymer, a polyvinyl acetate, a polyvinyl acetate-acrylate, a polyethylene glycol (PEG), a poloxamer and a mixture of two or thereof, preferably the hydrophilic polymer is polyvinylpyrrolidone; and or
the liquid formulation has a total amount of the hydrophilic polymer of 0.1 to 1.5 % w/v, preferably from 0.2 to 1.0 % w/v, more preferably from 0.3 to 0.7 % w/v.

7. The liquid formulation according to any one of claims 1 to 6, wherein the organic co-solvent is an alcohol, which is a monohydric alcohol having 2 to 6 carbon atoms, a polyhydric alcohol having 2 to 8 carbon atoms, a polyethylene glycol or a poloxamer preferably, wherein the organic co-solvent is propylene glycol, 1,3-propanediol, glycerol or a mixture of two or more thereof, more preferably wherein the organic co-solvent is propylene glycol or wherein the organic co-solvent is glycerol.

8. The liquid formulation according to anyone of claims 1 to 7, having a total amount of the organic co-solvent of from 2.5 to 60.0 % w/v, preferably from 5.0 to 55.0 % w/v, more preferably 7.5 to 25.0 % w/v.

9. The liquid formulation according to any one of claims 1 to 8, further comprising a preservative, a buffering agent, an antifoaming agent, a flavouring agent, a sweetener and a combination of two or more thereof.

10. The liquid formulation according to any one of claims 1 to 9, further comprising a flavouring agent.

11. A liquid formulation for use in therapy and/or for use as a medicament, wherein the liquid formulation is defined in any one of the preceding claims.

12. A liquid formulation for use in the treatment or prevention of pituitary-dependent hyperadrenocorticism or adrenal-dependent hyperadrenocorticism, wherein the liquid formulation is defined in any one of claims 1 to 10.

13. A liquid formulation for use as claimed in claim 12, in the treatment or prevention of pituitary-dependent hyperadrenocorticism or adrenal-dependent hyperadrenocorticism in a non-human animal, preferably a dog or a cat, more preferably a dog.

14. A kit of parts comprising (a) the liquid formulation according to any one of claims 1 to 10 and (b) a syringe.

## Patentansprüche

1. Flüssige Zubereitung, umfassend:
(a) Trilostan oder ein pharmazeutisch annehmbares Salz davon;
(b) einen Viskositätsmodifikator;
(c) ein organisches Co-Lösungsmittel; und
(d) Wasser;
wobei das organische Co-Lösungsmittel zumindest eine Hydroxygruppe aufweist und wasserlöslich oder wassermischbar ist.

2. Flüssige Zubereitung nach Anspruch 1, wobei der Viskositätsmodifikator ausgewählt ist aus der Gruppe bestehend aus einem Polysaccharid, einer Cellulose oder einem Cellulosederivat, einem hydrophilen Polymer, einem Siliciumdioxidderivat und einem Gemisch von zwei oder mehr davon, wobei optional der Viskositätsmodifikator ein Polysaccharid ausgewählt aus der Gruppe bestehend aus Xanthangummi, einem Carrageenan, Tragantgummi, Guargummi, Agar und einem Gemisch von zwei oder mehr davon beinhaltet, wobei bevorzugt das Polysaccharid Xanthangummi beinhaltet.

3. Flüssige Zubereitung nach Anspruch 1 oder 2, wobei der Viskositätsmodifikator ein Polysaccharid beinhaltet und die flüssige Zubereitung eine Gesamtmenge des Polysaccharids von 0,1 bis 1,5 Gew./Vol.-%, bevorzugt von 0,2 bis 1,0 Gew./Vol.-%, bevorzugter von 0,4 bis 0,8 Gew./Vol.-% aufweist.

4. Flüssige Zubereitung nach einem der Ansprüche 1 bis 3, wobei der Viskositätsmodifikator eine Cellulose oder ein Cellulosederivat ausgewählt aus der Gruppe bestehend aus Cellulose; einer Carboxymethylcellulose oder einem Salz davon; Ethylcellulose; Methylcellulose; einem Celluloseether; einer Hydroxyethylcellulose oder einem Salz davon; einer Hydroxypropylcellulose oder einem Salz davon; einer Hydroxypropylmethylcellulose oder einem Salz davon; und einem Gemisch von zwei oder mehr davon beinhaltet,
wobei bevorzugt die Cellulose mikrokristalline Cellulose ist.

5. Flüssige Zubereitung nach einem der Ansprüche 1 bis 4, wobei der Viskositätsmodifikator eine Cellulose und/oder ein Cellulosederivat beinhaltet, wobei:
die flüssige Zubereitung eine Gesamtmenge der Cellulose und/oder des Cellulosederivats von 0,05 bis 1,5 Gew./Vol.-%, bevorzugt von 0,1 bis 1,0 Gew./Vol.-% bevorzugter von 0,2 bis 0,6 Gew./Vol.-% aufweist; und/oder
der Viskositätsmodifikator eine Cellulose und ein Cellulosederivat in einem Massenverhältnis von 25:1 bis 1:5, bevorzugt einem Massenverhältnis von 15:1 bis 5:1, beinhaltet.

6. Flüssige Zubereitung nach einem der Ansprüche 1 bis 5, wobei der Viskositätsmodifikator ein hydrophiles Polymer beinhaltet, wobei;
das hydrophile Polymer ausgewählt ist aus der Gruppe bestehend aus Polyvinylpyrrolidon, einem Polyvinylpyrrolidon-Vinylacetat-Copolymer, einem Polyvinylacetat, einem Polyvinylacetat-Acrylat, einem Polyethylenglykol (PEG), einem Poloxamer und einem Gemisch von zwei oder davon, bevorzugt das hydrophile Polymer Polyvinylpyrrolidon ist; und/oder
die flüssige Zubereitung eine Gesamtmenge des hydrophilen Polymers von 0,1 bis 1,5 Gew./Vol.-%, bevorzugt von 0,2 bis 1,0 Gew./Vol.-%, bevorzugter von 0,3 bis 0,7 Gew./Vol.- % aufweist.

7. Flüssige Zubereitung nach einem der Ansprüche 1 bis 6, wobei das organische Co-Lösungsmittel ein Alkohol ist, der ein einwertiger Alkohol mit 2 bis 6 Kohlenstoffatomen, ein mehrwertiger Alkohol mit 2 bis 8 Kohlenstoffatomen, ein Polyethylenglykol oder ein Poloxamer ist, wobei bevorzugt das organische Co-Lösungsmittel Propylenglykol, 1,3-Propandiol, Glycerin oder ein Gemisch von zwei oder mehr davon ist, wobei bevorzugter das organische Co-Lösungsmittel Propylenglykol ist oder wobei das organische Co-Lösungsmittel Glycerin ist.

8. Flüssige Zubereitung nach einem der Ansprüche 1 bis 7 mit einer Gesamtmenge des organischen Co-Lösungsmittels von 2,5 bis 60,0 Gew./Vol.-%, bevorzugt von 5,0 bis 55,0 Gew./Vol.-%, bevorzugter von 7,5 bis 25,0 Gew./Vol.-%.

9. Flüssige Zubereitung nach einem der Ansprüche 1 bis 8, ferner umfassend ein Konservierungsmittel, ein Puffermittel, ein Antischaummittel, ein Aromamittel, einen Süßstoff und eine Kombination von zwei oder mehr davon.

10. Flüssige Zubereitung nach einem der Ansprüche 1 bis 9, ferner umfassend ein Aromamittel.

11. Flüssige Zubereitung zur Verwendung in Therapie und/oder zur Verwendung als ein Medikament, wobei die flüssige Zubereitung in einem der vorhergehenden Ansprüche definiert ist.

12. Flüssige Zubereitung zur Verwendung bei der Behandlung oder Prävention von hypophysenabhängigem Hyperadrenokortizismus oder nebennierenabhängigem Hyperadrenokortizismus, wobei die flüssige Zubereitung in einem der Ansprüche 1 bis 10 definiert ist.

13. Flüssige Zubereitung zur Verwendung wie in Anspruch 12 beansprucht, bei der Behandlung oder Prävention von hypophysenabhängigem Hyperadrenokortizismus oder nebennierenabhängigem Hyperadrenokortizismus bei einem nichtmenschlichen Tier, bevorzugt einem Hund oder einer Katze, bevorzugter einem Hund.

14. Kit von Teilen, umfassend (a) die flüssige Zubereitung nach einem der Ansprüche 1 bis 10 und (b) eine Spritze.

## Revendications

1. Formulation liquide comprenant :
(a) du trilostane ou un sel acceptable pharmaceutiquement de celui-ci ;
(b) un modificateur de viscosité ;
(c) un co-solvant organique ; et
(d) de l'eau ;
dans laquelle le co-solvant organique comporte au moins un groupe hydroxy et est soluble dans l'eau ou miscible à l'eau.

2. Formulation liquide selon la revendication 1, dans laquelle le modificateur de viscosité est choisi dans le groupe constitué par un polysaccharide, une cellulose ou un dérivé de cellulose, un polymère hydrophile, un dérivé de silice et un mélange de deux de ceux-ci ou plus, éventuellement dans laquelle le modificateur de viscosité comprend un polysaccharide choisi dans le groupe constitué par la gomme xanthane, un carraghénane, la gomme adragante, la gomme de guar, l'agar et un mélange de deux de ceux-ci ou plus, de préférence le polysaccharide comprend la gomme xanthane.

3. Formulation liquide selon la revendication 1 ou 2, dans laquelle le modificateur de viscosité comprend un polysaccharide, et la formulation liquide présente une teneur totale en polysaccharide de 0,1 à 1,5 % p/v, de préférence de 0,2 à 1,0 % p/v, idéalement de 0,4 à 0,8 % p/v.

4. Formulation liquide selon l'une quelconque des revendications 1 à 3, dans laquelle le modificateur de viscosité comprend une cellulose ou un dérivé de cellulose choisi dans le groupe constitué par la cellulose ; une carboxyméthylcellulose ou un sel de celle-ci ; l'éthylcellulose ; la méthylcellulose ; un éther de cellulose ; une hydroxyéthylcellulose ou un sel de celle-ci ; une hydroxypropylcellulose ou un sel de celle-ci ; une hydroxypropylméthylcellulose ou un sel de celle-ci ; et un mélange de deux de ceux-ci ou plus,
de préférence, dans laquelle la cellulose est de la cellulose microcristalline.

5. Formulation liquide selon l'une quelconque des revendications 1 à 4, dans laquelle le modificateur de viscosité comprend une cellulose et/ou un dérivé de cellulose, dans laquelle :
la formulation liquide présente une teneur totale en cellulose et/ou en dérivé de cellulose de 0,05 à 1,5 % p/v, de préférence de 0,1 à 1,0 % p/v, idéalement de 0,2 à 0,6 % p/v ; et/ou
le modificateur de viscosité comprend une cellulose et un dérivé de cellulose dans un rapport massique de 25:1 à 1:5, de préférence un rapport massique de 15:1 à 5:1.

6. Formulation liquide selon l'une quelconque des revendications 1 à 5, dans laquelle le modificateur de viscosité comprend un polymère hydrophile, dans laquelle ;
le polymère hydrophile est choisi dans le groupe constitué par la polyvinylpyrrolidone, un copolymère de polyvinylpyrrolidone et d'acétate de vinyle, un polyacétate de vinyle, un polyacétate de vinyle-acrylate, un polyéthylène glycol (PEG), un poloxamère et un mélange de deux de ceux-ci ou, de préférence le polymère hydrophile est la polyvinylpyrrolidone ; et/ou
la formulation liquide présente une teneur totale en polymère hydrophile de 0,1 à 1,5 % p/v, de préférence de 0,2 à 1,0 % p/v, idéalement de 0,3 à 0,7 % p/v.

7. Formulation liquide selon l'une quelconque des revendications 1 à 6, dans laquelle le co-solvant organique est un alcool, qui est un alcool monohydrique comportant 2 à 6 atomes de carbone, un alcool polyhydrique comportant 2 à 8 atomes de carbone, un polyéthylène glycol ou un poloxamère de préférence, dans laquelle le co-solvant organique est le propylène glycol, le 1,3-propanediol, le glycérol ou un mélange de deux de ceux-ci ou plus, idéalement dans laquelle le co-solvant organique est le propylène glycol ou dans laquelle le co-solvant organique est le glycérol.

8. Formulation liquide selon l'une quelconque des revendications 1 à 7, présentant une teneur totale en co-solvant organique de 2,5 à 60,0 % p/v, de préférence de 5,0 à 55,0 % p/v, idéalement de 7,5 à 25,0 % p/v.

9. Formulation liquide selon l'une quelconque des revendications 1 à 8, comprenant en outre un conservateur, un agent tampon, un agent antimousse, un agent aromatisant, un édulcorant et une combinaison de deux de ceux-ci ou plus.

10. Formulation liquide selon l'une quelconque des revendications 1 à 9, comprenant en outre un agent aromatisant.

11. Formulation liquide destinée à être utilisée en thérapie et/ou à être utilisée en tant que médicament, dans laquelle la formulation liquide est définie dans l'une quelconque des revendications précédentes.

12. Formulation liquide destinée à être utilisée dans le traitement ou la prévention d'un hyperadrénocorticisme d'origine hypophysaire ou d'un hyperadrénocorticisme d'origine surrénalienne, dans laquelle la formulation liquide est définie dans l'une quelconque des revendications 1 à 10.

13. Formulation liquide destinée à être utilisée selon la revendication 12, dans le traitement ou la prévention d'un hyperadrénocorticisme d'origine hypophysaire ou d'un hyperadrénocorticisme d'origine surrénalienne chez un animal non humain, de préférence un chien ou un chat, idéalement un chien.

14. Kit de pièces comprenant (a) la formulation liquide selon l'une quelconque des revendications 1 à 10 et (b) une seringue.
